# EUROPÄISCHE PATENTSCHRIFT

(11) **EP 4 098 364 B1**
(45) Veröffentlichungstag und Bekanntmachung des Hinweises auf die Patenterteilung: **29.11.2023**
(21) Anmeldenummer: 22173426.2
(22) Anmeldetag: 16.05.2022
(51) Int. Cl.: B01L 9/06, B01L 7/02, B01L 3/00, A61M 1/02

(54) **TEMPERIERVORRICHTUNG FÜR TEMPERIERGUT IN EINEM ZYLINDERFÖRMIGEN BEHÄLTNIS**
TEMPERING DEVICE FOR TEMPERING MATERIAL IN A CYLINDRICAL CONTAINER
DISPOSITIF DE THERMORÉGULATION POUR PRODUITS À THERMORÉGULER DANS UN RÉCIPIENT CYLINDRIQUE

(30) Priorität: 31.05.2021 DE 102021113984
(43) Veröffentlichungstag der Anmeldung: 07.12.2022
(73) Patentinhaber: Barkey GmbH & Co. KG, 33818 Leopoldshöhe (DE)
(72) Erfinder: Nowack, Armin, 33335 Gütersloh (DE); Barkey, Christian, 33619 Bielefeld (DE)
(74) Vertreter: Ostermann, Thomas

(56) Entgegenhaltungen:
- DE-A1-102018 112 194
- JP-A- 2006 325 751
- JP-A- 2020 115 813
- US-A1- 2004 053 186

## Beschreibung

Die Erfindung betrifft eine Vorrichtung zum Temperieren und Auftauen von einem in einem starren Behältnis angeordneten Temperiergut nach dem Oberbegriff des Patentanspruchs 1.

Aus der DE 10 2014 103 930 A1 ist eine Vorrichtung zum Lagern von zylinderförmigen Behältnissen, insbesondere Reagenzgläsern, bekannt, die beabstandet zueinander angeordnete Auflageleisten mit Ausnehmungen aufweist, so dass eine Vielzahl von Reagenzgläsern nebeneinander lagesicher in den Ausnehmungen gehalten ist. Die Auflageleisten bestehen aus einem starren Material.

Aus der DE 10 2018 112 194 A1 ist eine Vorrichtung zum Temperieren und Auftauen von in einem Behältnis angeordneten Temperiergut bekannt, die eine Heizeinrichtung zur Temperierung eines in einer Lagerkammer angeordneten Temperiergutes umfasst. Die Heizeinrichtung weist ein flächiges Heizelement auf, das zu beiden Seiten des das Temperiergut enthaltenen und als Beutel ausgebildeten Behältnisses mit demselben in Kontakt steht und durch Wärmeübertrag zu einem homogenen Auftauen des Temperiergutes führt. Die Größe der flächigen Heizelemente, die zu beiden Seiten des Temperiergutes angeordnet sind, sind an die Dimension des Temperiergutbeutels angepasst, so dass quasi der gesamte Beutel von den Heizelementen umgeben ist. Zusätzlich ist ein mechanisches Stellelement vorgesehen, mittels dessen der Temperiergutbeutel in vorzugsweise rhythmische Bewegung versetzt wird, was den Auftauvorgang beschleunigt. Ist das Temperiergut jedoch nicht in Beuteln, sondern in langgestreckten starren Behältnissen, beispielsweise Reagenzgläsern, enthalten, besteht die Gefahr, dass sich die Reagenzgläser während des Auftauvorgangs ihre vorgesehene Position zwischen den Heizelementen verlassen, insbesondere an den Rand der Lagerkammer bewegt werden, so dass das Auftauergebnis beeinträchtigt ist.

Aus der US 2004/053186 A1 ist eine Vorrichtung zum Temperieren und Auftauen eines in einem Behältnis angeordneten Temperaturgutes bekannt, wobei das kassettenartige Behältnis auf einer flexiblen wärmeübertragenden Platte aufliegt. Diese Matte weist keine Durchbrüche auf, so dass das Behältnis auf einer Oberseite der Matte aufliegt.

Aus der JP 2006 325751 A ist ein Wärmetauscher für Infusionslösungen bekannt, die einen Patienten zugeführt wird. Zur Temperierung des in Rohren fließende Temperiergutes weist der Wärmetauscher ein Peltier-Heizmodul auf. Zwischen dem Heizmodul und den Rohrbehältnissen ist ein Wärmeleitungsblock vorgesehen, der eine an die Form des Behältnisses angepasste Form aufweist, so dass die Mantelfläche des Behältnisses von den Flächen des Wärmeübertragungsblocks umschlossen sind. Die Behältnisse weisen somit keinen direkten Kontakt zu dem Heizmodul auf.

Aus der JP 2020 115813 A ist eine Vorrichtung zum Temperieren und Auftauen von einem in einem starren Behältnis angeordneten Temperiergut bekannt, wobei in einem Gehäuse eine Heizeinrichtung mit einem Heizelement und eine Lagermatte zur Aufnahme des Behältnisses vorgesehen sind.

Aufgabe der vorliegenden Erfindung ist es daher, eine Vorrichtung zum Temperieren und Auftauen von in einem starren Behältnis angeordneten Temperiergut derart weiterzubilden, dass ein homogenes Auftauen bzw. Temperieren des in dem Behältnis enthaltenen Temperiergutes gewährleistet ist.

Zur Lösung dieser Aufgabe weist die Erfindung die Merkmale des Patentanspruchs 1 auf.

Der besondere Vorteil der Erfindung besteht darin, dass durch das Lagern der starren Behältnisse auf einer Lagermatte ein positionsgenaues Lagern derselben gewährleistet ist bzw. ein unerwünschtes Verrutschen verhindert wird. Vorteilhaft kann hierdurch ein kontrolliertes und homogenes Auftauen des in den Behältnissen enthaltenen Temperiergutes erfolgen.

Nach der Erfindung sind die Behältnisse jeweils zylinderförmig ausgebildet. Die Lagermatte weist eine Mehrzahl von langgestreckten Aufnahmen mit Durchbrechungen auf, so dass das an einer Rückseite der Lagermatte angeordnete Heizelement in direkten Kontakt mit dem Behältnis treten kann. Der Wärmeübertrag kann somit verbessert werden.

Nach einer bevorzugten Weiterbildung der Erfindung besteht die Lagermatte aus einem flexiblen Material, so dass sie während des Auftau- und/oder Temperiervorganges die durch eine Bewegung des flächigen Heizelementes erzeugte Lageveränderung der Behältnisse folgen oder ausgleichen kann. Darüber hinaus kann durch die Flexibilität der Lagermatte der auf die Behältnisse wirkende Stoßvorgang abgemildert werden.

Nach einer Weiterbildung der Erfindung ist die Lagermatte aus einem Silikonmaterial, vorzugsweise aus einem Silikonkautschukmaterial, gebildet. Vorteilhat kann sich hierdurch die Lagermatte an die Lagerumgebung optimal anpassen.

Nach einer Weiterbildung der Erfindung ist der Lagermatte und/oder dem Heizelement ein mechanisch bewegbares Stellelement zugeordnet, mittels dessen das Heizelement und/oder die Lagermatte in eine periodische und/oder aperiodische Hin- oder Herbewegung verbringbar ist. Vorteilhaft kann die Lagermatte dieser Hin- und Herbewegung folgen und für ein sicheres Lagern der Behältnisse während des Auftau-/Temperiervorganges sorgen.

Nach einer Weiterbildung der Erfindung ist das mechanisch bewegbar Stellelement starr ausgebildet mit einer Mehrzahl von dünnen Streben, die vorzugsweise zu einem starren Rahmen geformt sind. Durch das Hin- und Herverschwenken des so gebildeten starren Rahmens kann unabhängig von der Aufheizmethode eine die das Temperieren bzw. Auftauen fördernde Bewegung der Behältnisse bewirkt werden, so dass das Temperieren bzw. Auftauen des Inhalts der Behältnisse beschleunigt wird.

Nach einer Weiterbildung der Erfindung ragen außenseitig der Lagermatte eine Anzahl von Haltelappen ab, die sich beispielsweise an einer aufrechten Seitenwand der Lagerkammer abstützen können, wenn die Dimension der Lagermatte größer ist als eine Grundfläche der Lagerkammer bzw. Auflagefläche des Gehäuses. Alternativ oder zusätzlich kann der Haltelappen auch zur teilweisen Überdeckung eines Zubehörteils dienen, was zur Bearbeitung der Behältnisse verwendet wird. In diesem Fall ist die Dimension der Lagermatte gleich oder kleiner als die Grundfläche der Lagerkammer.

Weitere Vorteile der Erfindung ergeben sich aus den weiteren Unteransprüchen.

Ausführungsbeispiele der Erfindung werden nachfolgend anhand der Zeichnungen näher erläutert.

Es zeigen:
- Fig. 1: einen schematischen Querschnitt durch eine Lagerkammer einer erfindungsgemäßen Vorrichtung,
- Fig. 2: eine Draufsicht auf eine Lagermatte, die nicht mit Behältnissen versehen ist,
- Fig. 3: einen Querschnitt durch eine alternative Lagermatte mit aufgelagerten Behältnissen und
- Fig. 4: eine Draufsicht auf die Lagermatte gemäß Figur 3 ohne aufgelagerte Behältnisse,
- Fig. 5: eine Draufsicht auf eine Lagermatte nach einer weiteren Ausführungsform und
- Fig. 6: eine Draufsicht auf ein starres Stellelement, mittels dessen die Lagermatte und/oder das Heizelement hin- und herbewegbar ist.

Eine erfindungsgemäße Vorrichtung zum Temperieren und/oder Auftauen von einem in langgestreckten starren Behältnissen 1 angeordneten Temperiergut 2 weist ein Gehäuse 3 auf, das in einem unteren Bereich eine Basiskammer 4 und in einem oberen Bereich eine Lagerkammer 5 aufweist. Die Behältnisse können beispielsweise als Vials (kleine Flaschen) für Proben oder dergleichen ausgeführt sein.

Die Lagerkammer 5 ist untenseitig und die Basiskammer 4 obenseitig durch eine starre Trennwand 6 begrenzt. Die Lagerkammer 5 ist topfförmig ausgebildet und weist aufrechte Seitenwände 7 auf, die eine obere Öffnung begrenzen. Die obere Öffnung der Lagerkammer 5 ist mittels eines verschwenkbaren Deckels 8 öffenbar und verschließbar.

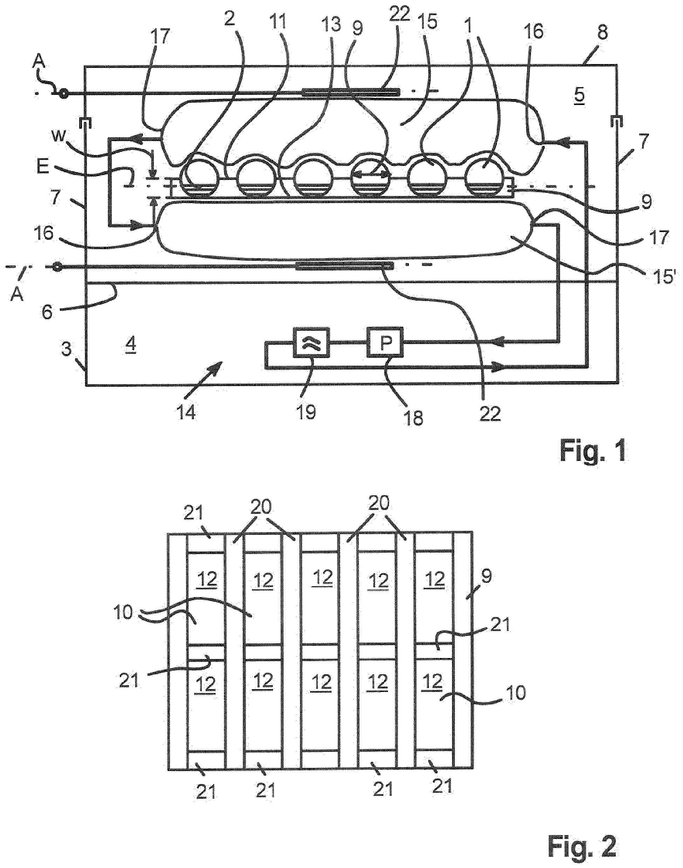

In der Lagerkammer 5 ist eine Lagermatte 9 zur positionsgenauen Lagerung bzw. Halterung einer Mehrzahl von langgestreckten Behältnissen 1, vorzugsweise zylinderförmigen Behältnissen, insbesondere Reagenzgläsern, vorgesehen. Zu diesem Zweck weist die Lagermatte 9 langgestreckte Aufnahmen 10 auf, so dass die zylinderförmigen Behältnisse 1 von einer Oberseite der Lagermatte 9 auf dieselbe ansetzbar sind. Die langgestreckten Aufnahmen 10 sind vorzugsweise als langgestreckte Ausnehmungen ausgebildet, so dass die Behältnisse 1 bezogen auf eine Oberseite 11 der Lagermatte 9 zumindest bereichsweise vertieft angeordnet sind. Nach einer ersten Ausführungsform der Lagermatte 9 gemäß den Figuren 1 und 2 weisen die langgestreckten Ausnehmungen 10 bereichsweise Durchbrüche 12, vorzugsweise langgestreckte Durchbrüche 12, auf, so dass die Behältnisse 1 in der Lagerstellung zumindest bündig zu einer Unterseite 13 der Lagermatte 9 anordbar sind, wie es in Figur 1 dargestellt ist, oder aus einer Ebene der Unterseite 13 herausragen.

Die Vorrichtung weist eine Heizeinrichtung 14 auf, die zwei flächige Heizelemente 15, 15' umfasst. Ein oberes Heizelement 15 ist oberhalb der mit den Behältnissen 1 versehenen Lagermatte 9 und ein unteres Heizelement 15' unterhalb der mit den Behältnissen 1 versehenen Lagermatte 9 angeordnet. Die beiden Heizelemente 15, 15' sind jeweils als Kunststoffbeutel ausgebildet mit einem Einlass 16 und mit einem Auslass 17, so dass ein temperiertes Medium, vorzugsweise ein sich in einem flüssigen Aggregatzustand befindliches Medium, die Heizelemente 15, 15' durchströmen kann. Zu diesem Zweck weist die Heizeinrichtung 14 eine Pumpe 18 sowie eine Heizquelle 19 auf, die vorzugsweise in der Basiskammer 4 angeordnet sind. Die Pumpe 18 und die Heizquelle 19 sind mit den Heizelementen 15, 15' über Schlauchleitungen verbunden. Vorzugsweise sind die Heizelemente 15, 15' in Strömungsrichtung hintereinander angeschlossen.

Wie aus Figur 2 zu ersehen ist, überstreichen die Durchbrüche 12 in Projektion auf eine Erstreckungsebene E der Lagermatte 9 eine größere Fläche als die Hälfte der entsprechend in die Erstreckungsebene E projizierten Fläche der langgestreckten Aufnahme 10. Vorzugsweise liegt die Fläche der Durchbrüche 12 in einem Bereich zwischen 50 % und 90% der projizierten Fläche der langgestreckten Aufnahme 10, so dass die Behältnisse 1 auf einer den unteren Heizelementen 15' zugewandten Seite mit einer größtmöglichen Fläche unmittelbar bzw. direkt an dem unterem Heizelement 15' anliegen. Es wird somit eine direkte Kontaktierung der Behältnisse 1 mit dem oberen Heizelement 15 und dem unteren Heizelement 15' erzielt, was die Wärmeeinleitung in die Behältnisse 1 beim Auftauen derselben verbessert.

Im ersten Ausführungsbeispiel gemäß den Figuren 1 und 2 ist eine Quererstreckung q der Behältnisse 1 größer als eine Wandstärke w der Lagermatte 9 in einem zu den langgestreckten Aufnahmen 10 benachbarten Zwischenbereich 20. In dem Zwischenbereich 20 erstreckt sich in einer ebenen Ausgangsstellung der Lagermatte 9 die Oberseite 11 parallel zu der Unterseite 13 derselben. Die in einem Aufnahmebereich der Lagermatte 9 angeordneten langgestreckten Aufnahmen 10 weisen eine Anzahl von axial versetzt zueinander angeordnete, an die Außenkontur der Behältnisse 1 angepasste Aufnahmeabschnitte 21 auf. Die Behältnisse 1 liegen auf den Aufnahmeabschnitten 21 der Aufnahmen 10 auf. Wenn die Behältnisse 1 im Querschnitt zylinderförmig ausgebildet sind, sind die Aufnahmeabschnitte 21 im Querschnitt kreisförmig.

Im vorliegenden Ausführungsbeispiel besteht die Lagermatte 9 aus einem flexiblen Material, vorzugsweise einem Silikonmaterial, insbesondere einem Silikonkautschukmaterial.

Zusätzlich kann in der Lagerkammer 5 ein mechanisch bewegbares Stellelement 22 angeordnet sein, mittels dessen das obere Heizelement 15 und/oder das untere Heizelement 15' in Bewegung versetzbar ist. Das mechanische Stellelement 22 kann beispielsweise als ein Paddelelement ausgebildet sein, das um eine Achse A periodisch und/oder aperiodisch hin- und herbewegbar ist und zwar in einem vorgegebenen spitzen oder stumpfen Winkelbereich. Die Drehachse A verläuft parallel zu der Erstreckungsebene E der Lagermatte 9 bzw. zu einer Erstreckungsebene der Heizelemente 15, 15'.

Eine nicht dargestellte Ansteuereinrichtung für das Stellelement 22 ist in der Basiskammer 4 angeordnet. Diese Steuereinrichtung kann auch zur Ansteuerung der Pumpe 18 und der Heizquelle 19 dienen, wenn die Heizquelle 19 als eine elektrisch betreibbare Heizquelle, beispielsweise Widerstandsheizung, ausgebildet ist.

Im vorliegenden Ausführungsbeispiel sind den Heizelementen 15, 15' jeweils ein Stellelement 22 zugeordnet, wobei das Stellelement 22 auf einer der Lagermatte 9 abgewandten Seite der Heizelemente 15, 15' angeordnet ist.

Nach einer nicht dargestellten alternativen Ausführungsform der Erfindung kann in der Lagerkammer 5 auch lediglich ein einziges Stellelement 22 angeordnet sein, das auf einer der Lagermatte 9 abgewandten Seite oder auf einer der Lagermatte 9 zugewandten Seite der Heizelemente 15, 15' an denselben angreift.

Nach einer alternativen Ausführungsform kann das Stellelement 22 direkt an einem Ende des oberen Heizelements 15 und/oder des unteren Heizelements 15' angeordnet sein, wobei die Hin- und Herbewegung nach oben und unten verläuft. Hierdurch wird das Ende des Heizelements 15, 15' periodisch und/oder aperiodisch nach oben und unten bewegt, so dass sich eine Wellenbewegung von diesem Punkt zu einem gegenüberliegenden Ende des Heizelements 15, 15' fortsetzt. Das Stellelement 22 kann fest mit dem Heizelement 15 bzw. 15' verbunden sein.

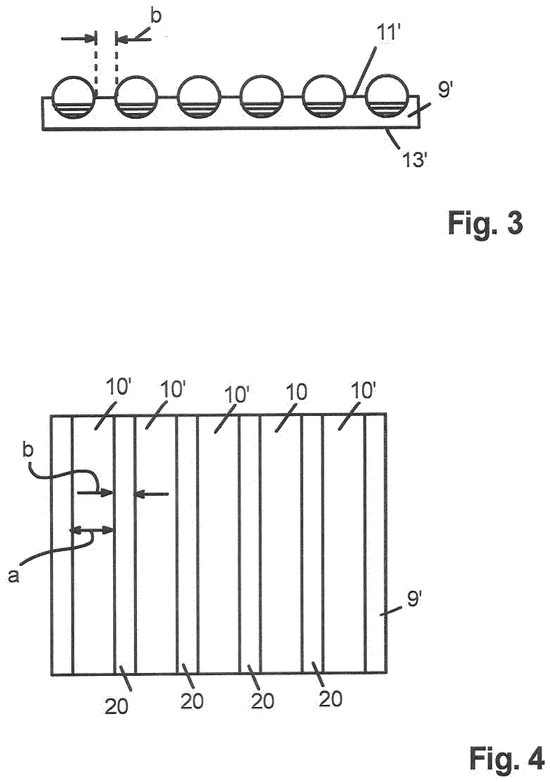

Nach einer alternativen Ausführungsform (nicht gemäß der Erfindung) entsprechend den Figuren 3 und 4 ist eine Lagermatte 9' vorgesehen, die im Unterschied zu der Lagermatte 9 keine Durchbrüche aufweist. Eine Unterseite 13' der Lagermatte 9' ist eben ausgebildet. Lediglich eine Oberseite 11' der Lagermatte 9' weist durchgehende langgestreckte Ausnehmungen 10' auf, in denen die Behältnisse 1 gelagert sind.

Gleiche Bauteile bzw. Bauteilfunktionen sind mit den gleichen Bezugsziffern versehen.

Die Lagermatte 9' weist zwischen den langgestreckten Ausnehmungen 10' Zwischenbereiche 20 auf, in denen die Oberseite 11' und die Unterseite 13' der in einer ebenen Lage befindlichen Lagermatte 9 parallel zueinander verlaufen.

Bei dieser Ausführungsform liegt die Unterseite 13' der Lagermatte 9' vollflächig an dem unteren Heizelement 15' an. Da die Lagermatte 9' aus einem flexiblen und wärmeleitenden Material besteht, kann die von dem unteren Heizelement 15' abgegebene Wärme an die Behältnisse 1 weitergegeben werden.

Nach einer nicht dargestellten alternativen Ausführungsform der Erfindung kann der Abstand b benachbarter langgestreckter Aufnahmen 10, 10' kleiner ausgebildet sein als eine Breite a der Ausnehmung 10, 10'. Zur Erhöhung der Packungsdichte der Behältnisse 1 auf der Lagermatte 9, 9' kann die Breite b des Zwischenbereichs 20 relativ klein gewählt werden, beispielsweise im Millimeterbereich.

Das Temperiergut 2 kann beispielsweise als ein flüssiges oder festes Gen- oder Stammzellenmaterial ausgebildet sein.

Nach einer alternativen Ausführungsform der Erfindung können die Heizelemente 15, 15' als Temperierkissen oder Gelkissen ausgebildet sein, die jeweils elektrisch erwärmt werden.

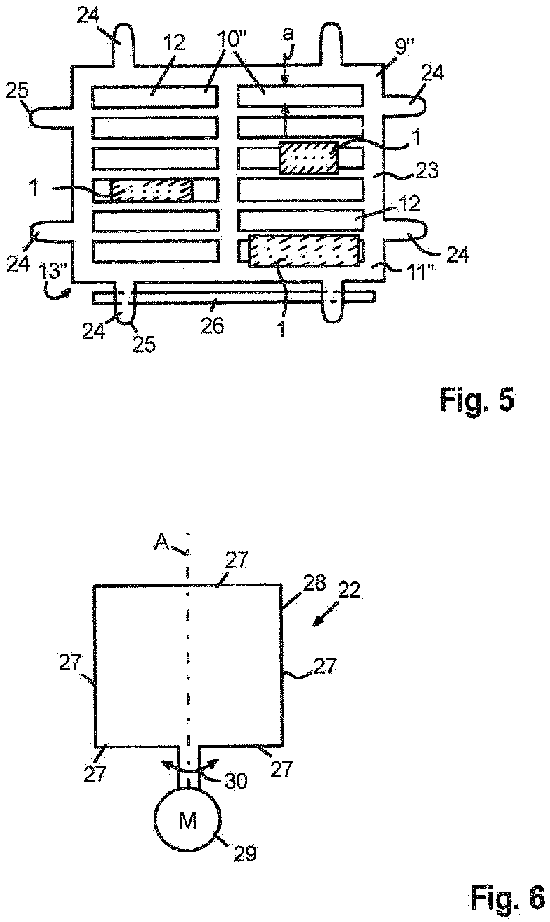

Nach einer weiteren Ausführungsform einer Lagermatte 9" gemäß Figur 5 weist diese einen umlaufenden Flachrahmen 23 auf, der eine Mehrzahl von rechteckig ausgeformten Ausnehmungen 10" umgibt. Der Flachrahmen 23 hat eine Breite im Bereich von 250 mm bis 300mm. Die langgestreckten Ausnehmungen 10" sind als Durchbrüche 12 ausgebildet und weisen jeweils eine Breite a im Bereich von 10 mm bis 50 mm auf. Die Breiten a der Ausnehmungen 10" sind vorzugsweise gleich ausgebildet.

Im vorliegenden Ausführungsbeispiel sind zwei versetzt zueinander angeordnete Reihen von jeweils sechs Ausnehmungen 10" vorgesehen. Auf einer Oberseite 11" der Lagermatte 9" werden die zu lagernden Behältnisse 1 abgelegt. Auf einer Außenseite des geschlossenen umlaufenden Flachrahmens 23 sind verteilt einer Anzahl von Haltelappen 24 angeordnet, deren freies Ende 25 im Wesentlichen senkrecht von dem Flachrahmen 23 nach außen hin abragen. Die Haltelappen 24 können zur Stützung der Lagermatte 9" bzw. der Behältnisse 1 an einem Rand des Gehäuses 3 und/oder an dem unteren Heizelement 15 dienen. Alternativ oder zusätzlich kann der Haltelappen 24 auch zur teilweisen Überdeckung eines Zubehörteils 26 dienen, das randseitig in der Lagerkammer 5 platziert ist.

Die Lagermatte 9" ist aus einem flexiblen Material, vorzugsweise aus einem Silikonmaterial, hergestellt.

Eine Wandstärke w der Lagermatte 9" liegt in einem Bereich von 1 mm bis 5 mm, vorzugsweise 2 mm. Die Ausnehmungen 10" weisen eine Dimension von 125 mm x 25 mm auf.

In Figur 6 ist die Dimension des mechanisch bewegbaren Stellelementes 22 erkennbar. Im Gegensatz zu der Lagermatte 9" ist das Stellelement 22 starr ausgebildet und weist eine Mehrzahl von senkrecht miteinander verbundenen dünnen Streben 27 auf. Die Streben 27 können im Querschnitt rechteckförmig oder kreisförmig ausgebildet sein. Vorzugsweise sind die Streben 27 aus einem massiven Material, vorzugsweise massivem Kunststoffmaterial, hergestellt. Die Streben 27 sind geradlinig ausgebildet.

Die Streben 27 bilden einen starren Rahmen 28, der vorzugsweise eine kleinere Dimension aufweist als der Flachrahmen 23 der Lagermatte 9". Alternativ oder zusätzlich können die Streben 27 auch bogenförmig ausgebildet sein, so dass der Rahmen 28 kreis- oder ellipsoidförmig in seiner Erstreckungsebene verläuft.

Der starre Rahmen 28 bzw. die Streben 27 werden mittels eines Aktuators 29, der beispielsweise als ein Elektro-Stellmotor ausgebildet sein kann, periodisch und/oder aperiodisch um einen Schwenkwinkel 30 hin- und herbewegt, wobei eine Schwenkachse bzw. Drehachse A eine Symmetrieachse des starren Rahmens 28 bildet. Die Drehachse A erstreckt sich parallel oder in der Erstreckungsebene E der Lagermatte 9".

Das Stellelement 22 kann - wie in Figur 1 dargestellt - auf einer der Lagermatte 9" abgewandten Seite des unteren Heizelementes 15' und/oder auf einer der Lagermatte 9" abgewandten Seite des oberen Heizelementes 15 angeordnet sein. Alternativ oder zusätzlich kann das Stellelement 22 auch zwischen dem oberen Heizelement 15 und der Oberseite 11" der Lagermatte 9" und/oder zwischen dem unteren Heizelement 15' und einer Unterseite 13" der Lagermatte 9" angeordnet sein.

## Patentansprüche

1. Vorrichtung zum Temperieren und Auftauen von einem in einem starren Behältnis (1) angeordneten Temperiergut (2) mit einem öffenbaren Gehäuse (3) enthaltend eine Lagerkammer (5) zur Aufnahme des Behältnisses (1), mit einer Heizeinrichtung (14) enthaltend ein flächiges Heizelement (15,15'), das sich in Wirkverbindung mit dem Behältnis (1) befindet, wobei zwischen dem flächigen Heizelement (15, 15') und dem Behältnis (1) eine Lagermatte (9, 9") zur Aufnahme des Behältnisses (1) angeordnet ist, **dadurch gekennzeichnet, dass** die Lagermatte (9, 9") eine Mehrzahl von langgestreckten Ausnehmungen (10, 10', 10") zur jeweiligen Aufnahme von zylinderförmig ausgebildeten Behältnissen (1) aufweist, wobei die langgestreckten Ausnehmungen (10, 10', 10") der Lagermatte (9, 9") mindestens einen Durchbruch (12) aufweisen.

2. Vorrichtung nach Anspruch 1, **dadurch gekennzeichnet, dass** die Lagermatte (9, 9") aus einem flexiblen Material, insbesondere aus einem einstückigen Silikonmaterial, besteht.

3. Vorrichtung nach Anspruch 1 oder 2, **dadurch gekennzeichnet, dass** das flächige Heizelement (15, 15') als ein ein temperiertes Medium enthaltener Kunststoffbeutel ausgebildet ist.

4. Vorrichtung nach Vorrichtung nach Anspruch 3, **dadurch gekennzeichnet, dass** der Kunststoffbeutel mit einer außerhalb der Lagerkammer (5) angeordneten Pumpe (18) und einer Heizquelle (19) wirkverbunden ist zur Temperierung des temperierten Mediums auf eine vorgegebene Temperatur.

5. Vorrichtung nach einem der Ansprüche 1 bis 4, **dadurch gekennzeichnet, dass** das temperierte Medium sich in einem flüssigen Aggregatzustand befindet.

6. Vorrichtung nach einem der Ansprüche 1 bis 5, **dadurch gekennzeichnet, dass** die Lagermatte (9) zwischen zwei flächigen Heizelementen (15, 15') angeordnet ist.

7. Vorrichtung nach einem der Ansprüche 1 bis 6, **dadurch gekennzeichnet, dass** die langgestreckten Ausnehmungen (10, 10', 10") der Lagermatte (9,9") eine Breite (a) im Bereich von 10 mm bis 50mm, aufweisen.

8. Vorrichtung nach einem der Ansprüche 1 bis 7, **dadurch gekennzeichnet, dass** die Lagermatte (9, 9") eine Anzahl von aus einer Erstreckungsebene (E) derselben hervorschwenkbaren Haltelappen (24) aufweist zum teilweisen Bedecken des Behältnisses (1) und/oder eines Zubehörteils (26).

9. Vorrichtung nach einem der Ansprüche 1 bis 8, **dadurch gekennzeichnet, dass** die Lagermatte (9, 9") einen umlaufenden Flachrahmen (23) aufweist, der die Mehrzahl von langgestreckten Ausnehmungen (10") umgibt und an dessen Außenseite die Mehrzahl von Haltelappen (24) angeordnet sind.

10. Vorrichtung nach einem der Ansprüche 1 bis 9, **dadurch gekennzeichnet, dass** ein freies Ende (25) des Haltelappens (24) sich senkrecht von dem Flachrahmen (23) der Lagermatte (9") anschließt.

11. Vorrichtung nach einem der Ansprüche 1 bis 10, **dadurch gekennzeichnet, dass** der Lagermatte (9) und/oder dem Heizelement (15, 15') ein mechanisch bewegbares Stellelement (22) zugeordnet ist, derart, so dass durch periodisches und/oder aperiodisches Bewegen des Stellelementes (22) die Lagermatte (9) in eine Hin- und Herbewegung quer zu einer Erstreckungsebene (E) derselben verbringbar ist.

12. Vorrichtung nach Anspruch 11, **dadurch gekennzeichnet, dass** das mechanisch bewegbare Stellelement (22) starr ausgebildet ist und eine Mehrzahl von miteinander verbundenen geraden und/oder gebogenen Streben (27) aufweist zur Bildung eines starren Rahmens (28), der eine kleinere Dimension aufweist als der Flachrahmen (23) der Lagermatte (9").

13. Vorrichtung nach Anspruch 11 oder 12, **dadurch gekennzeichnet, dass** das Stellelement (22) zwischen einem oberen Heizelement (15) und einer Oberseite (11") der Lagermatte (9, 9") oder auf einer der Lagermatte (9,9") abgewandten Seite des oberen Heizelementes (15) und/oder zwischen einem unteren Heizelement (15') und einer Unterseite (13') der Lagermatte (9") oder auf einer der Lagermatte (9") abgewandten Seite des unteren Heizelementes (15') angeordnet ist, wobei das Stellelement (22) um eine Schwenkachse (A) ansteuerbar ist, die parallel zur Erstreckungsebene (E) der Lagermatte (9") verläuft.

## Claims

1. A temperature-adjusting device for adjusting the temperature of and thawing a material (2) the temperature of which is to be adjusted and which is arranged in a rigid container (1), having an openable housing (3) containing a storage chamber (5) for receiving the container (1), having a heater (14) containing a flat heating element (15, 15') in operative connection with the container (1),
wherein a storage mat (9, 9") for receiving the container (1) is arranged between the flat heating element (15, 15') and the container (1), **characterized in that** the storage mat (9, 9") has a plurality of elongated recesses (10, 10', 10") for receiving individual cylindrically formed containers (1), wherein the elongated recesses (10, 10', 10") of the storage mat (9, 9") have at least one perforation (12).

2. The device according to Claim 1, **characterized in that** the storage mat (9, 9") is made of a flexible material, in particular an integral silicon material.

3. The device according to Claim 1 or 2, **characterized in that** the flat heating element (15, 15') is formed as a plastic pouch containing a temperature-adjusted medium.

4. The device according to Claim 3, **characterized in that** the plastic pouch is operatively connected to a pump (18) arranged outside the storage chamber (5) and to a heating source (19) for adjusting the temperature of the temperature-adjusted medium to a predetermined temperature.

5. The device according to any one of Claims 1 to 4, **characterized in that** the temperature-adjusted medium is in a liquid aggregate state.

6. The device according to any one of Claims 1 to 5, **characterized in that** the storage mat (9) is arranged between two flat heating elements (15, 15').

7. The device according to any one of Claims 1 to 6, **characterized in that** the elongated recesses (10, 10', 10") of the storage mat (9, 9") have a width (a) in the range of 10 mm to 50 mm.

8. The device according to any one of Claims 1 to 7, **characterized in that** the storage mat (9, 9") has a number of retaining lugs (24) pivotable out of an extension plane (E) thereof for partially covering the container (1) and/or a fitting (26).

9. The device according to any one of Claims 1 to 8, **characterized in that** the storage mat (9, 9") has a circumferential flat frame (23) surrounding the plurality of elongated recesses (10") and on the outer side of which the plurality of retaining lugs (24) are arranged.

10. The device according to any one of Claims 1 to 9, **characterized in that** a free end (25) of the retaining lug (24) attaches to the storage mat (9") perpendicularly from the flat frame (23).

11. The device according to any one of Claims 1 to 10, **characterized in that** the storage mat (9) and/or the heating element (15, 15') is associated with a mechanically movable positioning element (22) such that the storage mat (9) is able to be brought into a back-and-forth movement transverse to an extension plane (E) thereof through periodic and/or aperiodic movement of the positioning element (22).

12. The device according to Claim 11, **characterized in that** the mechanically movable positioning element (22) is rigidly formed and has a plurality of interconnected straight and/or bent struts (27) for forming a rigid form (28) having a smaller dimension than the flat frame (23) of the storage mat (9").

13. The device according to Claim 11 or 12, **characterized in that** the positioning element (22) is arranged between an upper heating element (15) and an upper side (11") of the storage mat (9, 9") or on a side of the upper heating element (15) facing away from the storage mat (9, 9") and/or between a lower heating element (15') and a lower side (13') of the storage mat (9") or on a side of the lower heating element (15') facing away from the storage mat (9"), wherein the positioning element (22) is actuatable about a pivot axis (A) running parallel to the extension plane (E) of the storage mat (9").

## Revendications

1. Dispositif d'ajustement de la température pour ajuster la température d'un produit (2) dont la température doit être ajustée et le décongeler, lequel produit étant agencé dans un récipient rigide (1), avec un boîtier (3) pouvant être ouvert, contenant une chambre de stockage (5) pour recevoir le récipient (1), avec un appareil de chauffage (14) contenant un élément chauffant plat (15, 15') qui se trouve en liaison fonctionnelle avec le récipient (1), un tapis de stockage (9, 9") pour recevoir le récipient (1) étant agencé entre l'élément chauffant plat (15, 15') et le récipient (1), **caractérisé en ce que** le tapis de stockage (9, 9") présente une pluralité d'évidements allongés (10, 10', 10") pour recevoir respectivement des récipients (1) réalisés sous forme cylindrique, les évidements allongés (10, 10', 10") du tapis de stockage (9, 9") présentant au moins un ajour (12).

2. Dispositif selon la revendication 1, **caractérisé en ce que** le tapis de stockage (9, 9") est constitué d'un matériau souple, notamment d'un matériau de silicone monobloc.

3. Dispositif selon la revendication 1 ou 2, **caractérisé en ce que** l'élément chauffant plat (15, 15') est réalisé sous la forme d'un sac en matière plastique contenant un milieu dont la température est ajustée.

4. Dispositif selon la revendication 3, **caractérisé en ce que** le sac en matière plastique est relié fonctionnellement à une pompe (18) agencée à l'extérieur de la chambre de stockage (5) et à une source de chauffage (19) pour ajuster la température du milieu dont la température est ajustée jusqu'à une température prédéfinie,

5. Dispositif selon l'une quelconque des revendications 1 à 4, **caractérisé en ce que** le milieu dont la température est ajustée se trouve à un état d'agrégation liquide.

6. Dispositif selon l'une quelconque des revendications 1 à 5, **caractérisé en ce que** le tapis de stockage (9) est agencé entre deux éléments chauffants plats (15, 15').

7. Dispositif selon l'une quelconque des revendications 1 à 6, **caractérisé en ce que** les évidements allongés (10, 10', 10") du tapis de stockage (9, 9") présentent une largeur (a) dans la plage allant de 10 mm à 50 mm.

8. Dispositif selon l'une quelconque des revendications 1 à 7, **caractérisé en ce que** le tapis de stockage (9, 9") présente un nombre de pattes de retenue (24) pouvant pivoter vers l'extérieur à partir d'un plan d'extension (E) de celui-ci pour recouvrir partiellement le récipient (1) et/ou un accessoire (26).

9. Dispositif selon l'une quelconque des revendications 1 à 8, **caractérisé en ce que** le tapis de stockage (9, 9") présente un cadre plat périphérique (23) qui entoure la pluralité d'évidements allongés (10") et sur le côté extérieur duquel sont agencées la pluralité de pattes de retenue (24).

10. Dispositif selon l'une quelconque des revendications 1 à 9, **caractérisé en ce qu'**une extrémité libre (25) de la patte de retenue (24) se raccorde perpendiculairement au cadre plat (23) du tapis de stockage (9").

11. Dispositif selon l'une quelconque des revendications 1 à 10, **caractérisé en ce qu'**un élément de réglage (22) mécaniquement déplaçable est associé au tapis de stockage (9) et/ou à l'élément chauffant (15, 15'), de telle sorte que, par déplacement périodique et/ou apériodique de l'élément de réglage (22), le tapis de stockage (9) peut être amené à effectuer un déplacement en va-et-vient transversalement à un plan d'extension (E) de celui-ci.

12. Dispositif selon la revendication 11, **caractérisé en ce que** l'élément de réglage (22) déplaçable mécaniquement est réalisé sous forme rigide et présente une pluralité d'entretoises (27) droites et/ou courbes reliées entre elles pour former un cadre rigide (28) qui présente une dimension plus petite que le cadre plat (23) du tapis de stockage (9").

13. Dispositif selon la revendication 11 ou 12, **caractérisé en ce que** l'élément de réglage (22) est agencé entre un élément chauffant supérieur (15) et un côté
supérieur (11") du tapis de stockage (9, 9") ou sur un côté de l'élément chauffant supérieur (15) détourné du tapis de stockage (9, 9") et/ou entre un élément chauffant inférieur (15') et un côté inférieur (13') du tapis de stockage (9") ou sur un côté de l'élément chauffant inférieur (15') détourné du tapis de stockage (9"), l'élément de réglage (22) pouvant être commandé autour d'un axe de pivotement (A) qui s'étend parallèlement au plan d'extension (E) du tapis de stockage (9").
